(19) Europäisches Patentamt
European Patent Office
Office européen des brevets

(11) Publication number: **0 001 287**
**B1**

(12) # EUROPEAN PATENT SPECIFICATION

(45) Date of publication of patent specification: 17.03.82

(21) Application number: 78100953.5

(22) Date of filing: 21.09.78

(51) Int. Cl.³: C 12 P 17/00, A 61 K 35/66, C 07 D 333/32 //C12R1/365

(54) **Antibiotic No. 2-200, process for its production and pharmaceutical compositions containing Antibiotic No. 2-200.**

(30) Priority: 22.09.77 JP 113205/77

(43) Date of publication of application: 04.04.79 Bulletin 79/7

(45) Publication of the grant of the patent: 17.03.82 Bulletin 82/11

(84) Designated Contracting States: DE FR GB

(56) References cited: None

(73) Proprietor: Chugai Seiyaku Kabushiki Kaisha
5-1, 5-chome, Ukima Kita-ku
Tokyo (JP)

(72) Inventor: Oishi, Hideo
214-42, Kitairiso Sayama-shi
Saitama-ken (JP)
Inventor: Noto, Takao
2200, 3-4-304, Yamazakicho Machida-shi
Tokyo (JP)
Inventor: Nawata, Yoshiharu
2-220-9, Suzukicho Kodaira-shi
Tokyo (JP)
Inventor: Okazaki, Hiroshi
1061-40, Minamiiriso Sayama-shi
Saitama-ken (JP)
Inventor: Sasaki, Hiroshi
7-8-9-403, Shimosato Higashikurume-shi
Tokyo (JP)
Inventor: Ando, Kunio
1877, Shimosakunobe Kawasaki-shi
Kanagawa-ken (JP)
Inventor: Ogawa, Haruki
4-14-17, Nishitsutsujigaoka Chofu-shi
Tokyo (JP)

(74) Representative: Vossius, Volker, Dr. et al,
Vossius, Vossius, Tauchner, Heunemann, Rauh
Patentanwälte Siebertstrasse 4 P.O. Box 86 07 67
D-8000 München 86 (DE)

EP 0 001 287 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Antibiotic No. 2-200, process for its production and pharmaceutical compositions containing Antibiotic No. 2-200

This invention relates to a novel antibiotic No. 2-200, a process for its production and pharmaceutical compositions containing said antibiotic.

As a result of searching for novel antibiotics produced by microorganisms, the present inventors have found that a certain actinomycete produces and accumulates an antibiotic that strongly inhibits the growth of a mutant of Pseudomonas aeruginosa, for example, G-75, M-57740, that is sensitive to β-lactam antibiotics. The present inventors continued their studies and succeeded in isolating from a culture of an actinomycete a substance in a crystalline form, which was subjected to X-ray diffractometry to determine its structure. The substance was found to be a novel antibiotic, which was designated No. 2-200. This invention is the result of further studies based on these findings.

This invention therefore relates to an antibiotic No. 2-200 of the formula

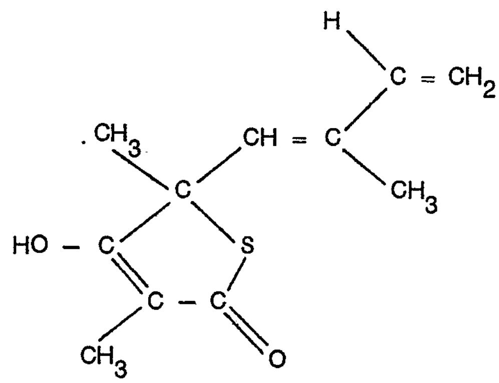

Furthermore the invention relates to a process for producing said antibiotic by cultivating in a nutrient medium the antibiotic No. 2-200 producing microorganism Nocardia sp. No. 2-200 ATCC 31319 and recovering the accumulated antibiotic No. 2-200 from the culture broth.

Finally the invention relates to pharmaceutical compositions containing antibiotic No. 2-200.

The microorganism of the genus Nocardia having the ability to produce No. 2-200 is the Nocardia sp. No. 2-200 strain isolated from a soil sample in Saitama Prefecture. The strain has been deposited on August 23, 1977 with ATCC under the identification number ATCC 31319 and also deposited at the Agency of Industrial Science and Technology, Research Institute under FERM-P-No. 4171.

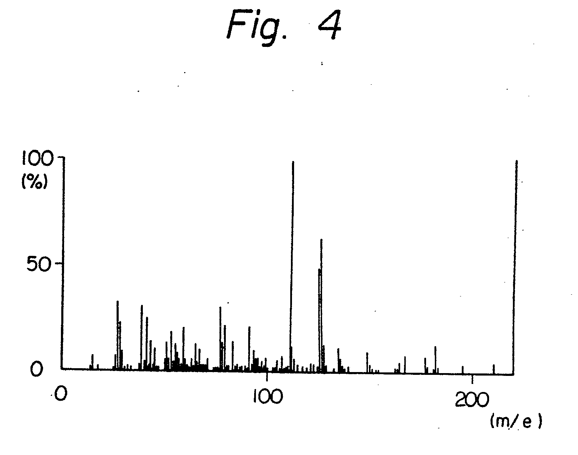

Fig. 1 shows the infrared absorption spectrum of the antibiotic No. 2-200; Fig. 2 shows the nuclear magnetic resonance spectrum of said antibiotic; Fig. 3 shows the ultraviolet absorption spectrum of the antibiotic; and Fig. 4 shows the mass spectrum of said antibiotic.

The strain Nocardia sp. No. 2-200 has the following characteristics:

(1) Morphological characteristics

The substrate mycelium is branched into short aerial mycelia. Colonies without aerial mycelia were yellowish brown and turned white as they developed aerial mycelia. Each aerial mycelium divided into short rods in the course of incubation; for instance, most of the aerial mycelia of cells incubated on a sucrose nitrate agar medium at 28°C for 21 days divided into bacillus-like cells. No sporophores as found in Streptomyces sp. were observed. Almost all substrate mycelia were pleomorphic and divided into rods.

From the above morphological features, it was concluded that the strain belongs to the genus Nocardia of soft type.

(2) Observation on various media (after incubation at 28°C for 18 days)

| Medium | Growth | Color of colony surface (Aerial mycelium) | Substrate mycelium | Soluble pigment and others |
|---|---|---|---|---|
| Trypton yeast extract (IPS-1) | moderate; part of colony precipitated, lens-like colony on liquid surface | | white | none |
| yeast malt extract agar (IPS-2) | good; elevated wrinkled | white, a few aerial mycelia | pale yellow orange | none |
| oatmeal agar (IPS-3) | moderate; flat | white | pale yellow orange | none |
| starch inorganic salt agar (IPS-4) | weak to moderate | white | pale yellowish brown | none; starch highly hydrolyzed |
| glycerol asparagine agar (IPS-5) | weak; powdery | white | pale red orange | none |
| peptone yeast extract iron agar (IPS-6) | moderate; elevated wrinkled | no aerial mycelium | pale yellowish brown | none |
| tyrosine agar (IPS-7) | good; powdery | white | pale yellowish brown | none |
| sucrose sodium nitrate agar (Waksman-1) | moderate | no aerial mycelium in the center of colony; white periphery | pale yellowish brown | none |
| glucose asparagine agar (Waksman-2) | good powdery | white | pale yellow orange | none |
| nutrient agar (Waksman-8) | moderate, | a few aerial mycelia, white | colorless | none |
| gelatin (Waksman-19) | moderate; wrinkled precipitate | colorless | — | none; strong gelatin liquefaction |

| Medium | Growth | Color of colony surface (Aerial mycelium) | Substrate mycelium | Soluble pigment and others |
|---|---|---|---|---|
| skimmed milk (Waksman-41) | weak | — | — | none; no coagulation or peptonization |

(3) Physiological properties

| | |
|---|---|
| Hydrolysis of starch | + |
| Tyrosinase reaction | — |
| Hydrolysis of gelatin | + |
| Coagulation and Peptonization of milk | — |
| Formation of $H_2S$ | — |

(4) Utilization of various carbohydrates (on IPS-9 medium)

| | | | | | |
|---|---|---|---|---|---|
| D-glucose | + | D-xylose | - | D-fructose | - |
| L-arabinose | ± | Inositol | - | Rhamnose | - |
| Sucrose | + | D-mannitol | - | Raffinose | - |

Like other species of the genus Nocardia, Nocardia sp. No. 2-200 easily undergoes a change in its characteristics; for example, it mutates easily by artificial mutating means such as ultraviolet rays, X-rays, radioactive rays or chemical mutagenic agents.

X-ray diffractometry shows that the antibiotic No. 2-200 of this invention has the following structure:

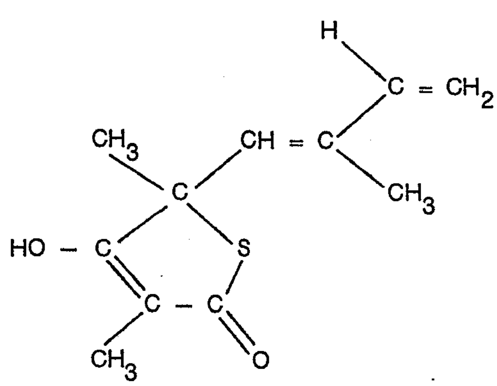

The physico-chemical properties of the antibiotic are as follows:

(1) Elementaltal analysis

| | C | H | S |
|---|---|---|---|
| Calculated (%) | 62.86 | 6.67 | 15.23 |
| Found (%) | 62.57 | 6.78 | 14.93 |

(2) Molecular formula

$C_{11}H_{14}O_2S$ with a molecular weight of 210 as determined by mass spectrometric analysis

(3) Melting point

124° to 126°C

(4) Infrared absorption spectrum (potassium bromide disc)

The antibiotic has an infrared absorption spectrum as shown in Fig. 1. The more characteristic absorption bands are set forth in the table below.

| | | |
|---|---|---|
| 3400 $cm^{-1}$ brd M | 2960 $cm^{-1}$ brd M | 2925 $cm^{-1}$ M |
| 1690 $cm^{-1}$ M | 1605 $cm^{-1}$ brd VS | 1445 $cm^{-1}$ M |
| 1396 $cm^{-1}$ M | 1380 $cm^{-1}$ M | 1365 $cm^{-1}$ M |
| 1323 $cm^{-1}$ VS | 1280 $cm^{-1}$ S | 1229 $cm^{-1}$ M |
| 1169 $cm^{-1}$ M | 1090 $cm^{-1}$ S | 1068 $cm^{-1}$ M |
| 1046 $cm^{-1}$ S | 1018 $cm^{-1}$ M | 987 $cm^{-1}$ M |
| 966 $cm^{-1}$ W | 927 $cm^{-1}$ W | 900 $cm^{-1}$ M |

Abbreviations: VS = very strong; S = strong; M = medium; W = weak; VW = very weak; brd = broad

(5) Nuclear magnetic resonance spectrum

The antibiotic has a nuclear magnetic resonance spectrum as shown in Fig. 2. The spectrum was obtained by spectrometry at 60 MHz using a 10% solution of the antibiotic in deutero methanol, with tetramethyl silane (TMS) added as the internal standard.

(6) Ultraviolet absorption spectrum

The antibiotic has an ultraviolet absorption spectrum as shown in Fig. 3. Methanol was used as the solvent. An absorption peak was observed at 237 nm and 300 nm under neutral conditions.

(7) Mass spectrum

The antibiotic has a mass spectrum as shown in Fig. 4. The peak m/e 111 is the base peak and the peak m/e 210 is the parent peak.

(8) Color reaction

| | |
|---|---|
| Discoloration of $KMnO_4$ | positive |
| Nitroprusside reaction | positive |
| Molisch reaction | negative |

(9) Solubility in solvents

| *Solvent* | *Solubility* |
|---|---|
| neutral to acidic water | slightly soluble |
| alkaline water | easily soluble |
| methanol | easily soluble |
| acetone | easily soluble |
| ethyl acetate | easily soluble |

(10) Rf on thin layer chromatography (using Kieselgel 60 F254, Art 5729, manufactured by E. Merck)

| *Solvent system* | *Rf* |
|---|---|
| benzene:acetone (3:1) | 0.3 |
| acetone | 0.8 |
| ethyl acetate | 0.5 |

The antibiotic No. 2-200 is an acidic substance which easily forms a nontoxic salt with an organic or inorganic base, such as ethanol amine, triethyl amine, caustic soda, caustic potash or ammonia. These salts are water soluble, and so, the antibiotic is advantageously used as an injectable preparation.

The novel antibiotic No. 2-200 of this invention is produced by cultivating *Nocardia* sp. No. 2-200 in a liquid nutrient medium under aerobic conditions, preferably submerged conditions, as described hereinafter.

According to the process of this invention, either a solid or liquid nutrient medium may be used for cultivating Nocardia sp. No. 2-200 so as to produce and accumulate the antibiotic No. 2-200 in the medium. Liquid cultivation may be performed e.g. by stationary cultivation or with the medium being stirred or agitated. Agitating the medium may be performed e.g. by aeration. For large scale production the culturing under aeration and stirring is preferred.

The composition of the nutrient medium is that conventionally used for the cultivation of actinomycetes. For instance, carbohydrates such as starch and glucose, alcohols such as glycerol, methanol and ethanol, aliphatic acids such as acetic acid, linoleic acid and palmitic acid, and vegetal and animal oils, such as soybean oil, cotton-seed oil and fish oil, may be used singly or in a mixture as a carbon source. Organic nitrogen sources such as soybean powder, cottonseed powder, corn steep liquor (hereinunder C.S.L.), yeast and meat extract, or inorganic nitrogen sources such as inorganic ammonium salts, for example ammonium sulfate, ammonium nitrate, ammonium chloride and ammonium phosphate may be used. Inorganic salts usually required for cultivation of actinomycetes such as calcium carbonate, sodium chloride, sodium nitrate, potassium chloride, potassium primary phosphate and sodium secondary phosphate may be used alone or in proper combination. If desired, heavy metal salts, vitamins as well as anti-foaming agents or surface active agents, such as silicone oil or polyalkylene glycol ether, may be used.

According to the process of this invention, the medium containing these nutrient components is inoculated with Nocardia sp. No. 2-200 for cultivation. The cultivation temperature is properly selected from the optimum temperature range for the growth of the Nocardia sp. strain, No. 2-200; it is normally from 15 to 35°C, preferably from 24 to 32°C. Cultivation may continue until maximum accumulation of the antibiotic No. 2-200 is obtained; normally, such period ranges from 12 to 168 hours, preferably from 24 to 144 hours.

The antibiotic No. 2-200 thus produced in the culture is isolated and recovered by conventional methods employed for recovering the metabolic product produced by a microorganism. The antibiotic of this invention is acidic and can be recovered by controlling the pH of the filtrate so as to change the partition coefficient between water and an organic solvent immiscible with water such as ethyl acetate or ether. More specifically, Nocardia sp. No. 2-200 is isolated from the culture filtrate that contains a major portion of the antibiotic No. 2-200; the filtrate is adjusted to pH 3 with a mineral acid, for example, hydrochloric acid, and extracted once with an equal amount of ethyl acetate, upon which the antibiotic No. 2-200 is transferred to the ethyl acetate layer. Vacuum concentration of the ethyl acetate layer produces an antibiotic-containing syrup. The syrup thus obtained is purified by column chromatography on Silica Gel G (product of E. Merck) and Sephadex (Trade Mark) LH-20 (product of Pharmacia) using Pseudomonas aeruginosa (G-75, M 57740 as the test organism. The antibiotic No. 2-200 is obtained in the form of pale yellow needles.

As shown in Table I, the antibiotic No. 2-200 exhibits strong antibacterial activity against gram negative bacteria. One significant advantage is that it exhibits particularly strong antibacterial activity against bacteria of genus Salmonella and Serratia. The antibiotic has an extremely weak toxicity and exhibits no delayed toxicity in mammals. Therefore, the antibiotic No. 2-200 of this invention is a valuable pharmaceutical for curing or preventing diseases due to infection with gram negative bacteria in humans or animals.

TABLE I

Minimum Inhibitory Concentration (MIC) for Gram-negative Bacteria

| Microorganism | MIC (μg/ml) No. 2-200 | MIC (μg/ml) Ceftezole |
|---|---|---|
| *Bacteroides fragilis* V-6 | 3.12 | 100 |
| *Bacteroides fragilis* V-7 | 12.5 | 25 |
| *Bacteroides fragilis* V-8 | 6.25 | >100 |
| *Fusobacterium glutinosum* 1006 | 100 | 6.25 |
| *Fusobacterium necroforum* S-45 | 3.12 | — |

Note: incubated at 37°C for 40 hours on GAM agar medium (product of Nippon Seiyaku) using anaerobic incubator. Inoculum size $10^6$/ml.

The antibiotic of this invention can be confectionated in a wide variety of oral or parenteral dosage forms, alone or in admixture with other co-acting substances. The pharmaceutical compositions may be a mixture of 0.01 to 99% of the antibiotic with a pharmaceutical carrier or carriers which can be a solid or liquid material in which the antibiotic is soluble, dispersible or suspensible. They can be prepared in a unit dosage form. The solid compositions can have the form of tablets, powders, dry syrups, troches, granules, capsules, pills, suppositories, or of similar solid preparations. The liquid compositions can be in form of injectable preparations, ointments, dispersions, inhalants, suspensions, solutions, emulsions, syrups, or elixirs. Any diluent (e.g. starch, sucrose, lactose, calcium carbonate, kaolin); bulking agent (e.g. lactose, sugar, salt, glycine, starch, calcium carbonate, calcium phosphate, kaolin, bentonite, talc, sorbitol); binder (e.g. starch, acacia, gelatin, glucose, sodium alginate, tragacanth, carboxymethylcellulose, sorbitol, polyvinylpyrrolidone); disintegrating agent (e.g. starch, agar, carbonates, sodium laurylsulfate), lubricant (e.g. stearic acid, talc, paraffin, boric acid, silica, sodium benzoate, polyethylene glycol, cacao oil, magnesium sulfate); emulsifying agent (e.g. lecithin, sorbitan monooleate, acacia); suspending agent (e.g. sorbitol, methylcellulose, glucose, sugar syrup, gelatin, hydroxyethylcellulose, carboxymethylcellulose, aluminum stearate gel, hydrogenated fats); solvent (e.g. water, peanut oil, sesame oil, methyl oleate); preservative (e.g. methyl or ethyl p-hydroxybenzoate, sorbic acid), edible coloring agent, aroma substance, solubilizing agent, buffer, stabilizer, dispersing agent, wetting agent, antioxidant, and other conventional auxiliary agents can be used in the conventional manner as far as they do not act adversely on the antibiotic.

The antibiotic of this invention can be used as a solution for intravenous, intramuscular, or subcutaneous injection. The antibiotic can be dissolved in an ampoule in an oily solvent for injection to give an injectable solution; in order to preserve the injectable preparation for a long period of time, it is appropriate to make a vial preparation containing crystals, powder, microcrystals, or lyophilizate of the antibiotic. The vial preparation may be dissolved or suspended in a solvent for injection immediately before use. The preparation may contain a preservative.

Furthermore, the antibiotic of this invention can be used as a suppository, ointment for topical use, powder for topical use, and a similar preparation preparable according to methods well-known to those skilled in the art. The preparation for topical use can contain 0.01 to 99% of the antibiotic of this invention together with a necessary amount of pharmaceutical carrier given above.

Example 1

100 ml of a nutrient medium comprising 3% of glucose, 1% of dry bouillon (product of Eiken Kagaku), 0.2% of yeast extract and 0.2% of calcium carbonate in water was placed in 500 ml conical flasks and sterilized. The medium was inoculated with spores of Nocardia sp. No. 2-200 (deposited at Agency of Industrial Science and Technology, Research Institute, under the identification number FERM-P-No. 4171) and cultivated on a rotary shaker at 28°C for 4 days. Thereafter the contents of the flasks were pooled. 400 ml of the seed culture obtained were transferred into a 30 liter stainless steel jar fermentor charged with 20 l of a sterile medium comprising 1% of glucose, 2% of starch, 2% of C.S.L., 0.1% of dry yeast and 0.2% of calcium carbonate (pH 6.5 before sterilization) in water, and cultivated at 28°C for 72 hours with agitation (400 rpm). Air was supplied at a rate of 10 l/min.

## Example 2

Nocardia sp. No. 2-200 was incubated in a liquid medium in the same manner as used in Example 1. The microorganism was isolated from the medium using a centrifugal dehydrator precoated with Radiolite-800 (product of Showa Kagaku). About 18 l of the filtrate was adjusted to pH 3 with dilute hydrochloric acid. The filtrate thus controlled for pH was intimately admixed with an equal amount of ethyl acetate to extract the antibiotic No. 2-200. The turbid mixture was filtered with Radiolite-800 as filter aid, and the filtrate was left standing until it separated into two layers. The lower water layer was discarded whereas the upper ethyl acetate layer was recovered, dehydrated with anhydrous sodium sulfate and concentrated in vacuo to obtain an oily substance that contained the antibiotic No. 2-200.

## Example 3

An oily substance produced by repeating the procedure of Example 2 was added to the top of a column prepared by suspending 200 g of Silica Gel G in benzene. The column was eluted with 2 l of a mixture of benzene and acetone (95:5) to remove impurities. The column was further eluted with 2 l of a mixture of benzene and acetone (9:1). The eluate was recovered in 15 ml fractions. The fractions having antibacterial activity against Pseudomonas aeruginosa G-75, M 57740 were combined and concentrated to give 7 g of an oily crude antibiotic No. 2-200.

## Example 4

An oily substance containing the antibiotic No. 2-200 produced by repeating the procedure of Example 3 was added to the top of a column prepared by suspending 75 g of Sephadex (Trade Mark) LH-20 in a benzene/acetone mixture (9:1). The column was eluted with 4 l of the same solvent. The eluate was recovered in 15 ml fractions and the fractions exhibiting antibacterial activity against Pseudomonas aeruginosa G-75, M 57740 were combined and concentrated in vacuo. The pale yellow oily product obtained was dried in vacuo in a desiccator to produce a crude crystalline product. The crystals were dissolved in a mixture of benzene and acetone (9:1) under heating and the solution left in a refrigerator. The yield of crystals was 2 g.

## Example 5

The crystals of the antibiotic No. 2-200 obtained in Example 4 had an antibacterial spectrum as shown in Table II. The test organisms were incubated by the agar plate dilution method at 37°C for 24 hours, after which their growth was observed. The test organisms were incubated on an NB medium (product of Eiken Kagaku) at 37°C overnight, and each culture obtained was diluted $10^3$- to $10^4$-fold and inoculated on an agar plate. The following agar plate media were used:

NA:
nutrient agar (product of Eiken Kagaku)

NA+G6P:
glucose-6-phosphoric acid (50 μg/ml) plus nutrient agar

Anti-3A:
antibiotic medium No. 3 agar (product of Difco)

MHA:
Mueller Hinton Agar (product of Eiken Kagaku)

TABLE II
Antibacterial Spectrum of Antibiotic No. 2-200

| Test organism | Minimum Inhibitory Concentration (uμg/ml) NA | NA+G6P | Anti-3A | MHA |
|---|---|---|---|---|
| *Bacillus subtilis* PCI-219 | 50 | 50 | 50 | 50 |
| *Bacillus cereus* | 50 | 50 | 50 | 50 |
| *Bacillus thuringiensis* | 50 | 25 | 50 | 50 |
| *Sarcina lutea* | 12.5 | 12.5 | 12.5 | 25 |
| *Staphylococcus epidermidis* TO-3 | 50 | 50 | 100 | 50 |
| *Staphylococcus aureus* 209P | >50 | ≧50 | >25 | 50 |
| *Staphylococcus aureus* 222 | >400 | ≧400 | >400 | 100 |
| *Staphylococcus aureus* JU-5 | >400 | ≧400 | >400 | 100 |
| *Staphylococcus aureus* A-5 | 50 | 25 | 25 | 50 |
| *Escherichia coli* NIHJ | 200 | 100 | 200 | 50 |
| *Escherichia coli* No. 9 | 50 | 50 | 100 | 50 |
| *Salmonella enteritidis* T-1 | 6.25 | 6.25 | 3.12 | 3.12 |
| *Salmonella typhi* TANAKA | 12.5 | 12.5 | 12.5 | 3.12 |
| *Salmonella paratyphi* A | 50 | 50 | 50 | 3.12 |
| *Klebsiella pneumoniae* 3K25 | 200 | 100 | 50 | 50 |
| *Klebsiella pneumoniae* 15c | 100 | 100 | 50 | 12.5 |
| *Shigella flexneri* 2b | 6.25 | 6.25 | 3.12 | 1.56 |
| *Shigella sonnei* | 100 | 100 | 100 | 50 |
| *Serratia marcescens* FU-111 | 50 | 50 | 25 | 6.25 |
| *Serratia marcescens* T-50 | 50 | 50 | 12.5 | 1.56 |
| *Pseudomonas aeruginosa* J-272 | >400 | >400 | >400 | >400 |

TABLE II — continued
Antibacterial Spectrum of Antibiotic No. 2-200

| Test organism | Minimum Inhibitory Concentration (uμg/ml) NA | NA+G6P | Anti-3A | MHA |
|---|---|---|---|---|
| *Pseudomonas aeruginosa* G-75 | 6.25 | 6.25 | 6.25 | 6.25 |
| *Pseudomonas aeruginosa* M57740 | 1.56 | 1.56 | 1.56 | ≥0.78 |
| *Proteus mirabilis* 1287 | 50 | 50 | 50 | 50 |
| *Proteus mirabilis* 9' | 50 | 50 | 100 | 200 |

Example 6

The crystals of the antibiotic No. 2-200 obtained in Example 4 were suspended in sterile distilled water, mixed with 5% caustic soda to adjust the pH to 7.0, and dissolved to give a solution. The solution was administered to 5-week-old ddY male mice to examine its acute toxicity as well as delayed toxicity. During the 10 days that followed the administration, the weight of each mouse, its food intake and water intake were measured. Acute toxicity ($LD_{50}$) was observed after administration of a single dose of 705 mg/kg i.v. and after intraperitoneal injection of 810 mg/kg. The antibiotic did not exhibit any delayed toxicity.

## Claims

1. An antibiotic No. 2-200 of the formula

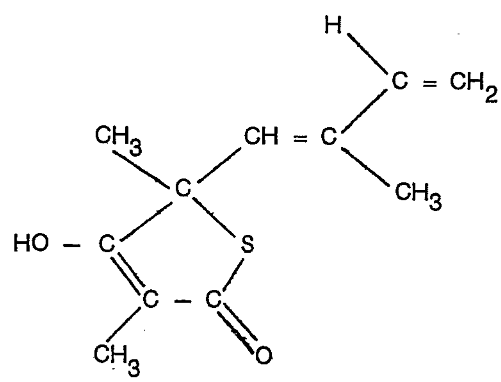

2. A process for producing an antibiotic No. 2-200 by cultivating in a nutrient medium the antibiotic No. 2-200 producing microorganism Nocardia sp. No. 2-200, ATCC 31319 and recovering the accumulated antibiotic No. 2-200 from the culture broth.

3. A process for producing an antibiotic No. 2-200 according to Claim 2, wherein the cultivation is performed at a temperature in the range of from 15 to 35°C for a period of 12 to 168 hours.

4. A process for producing an antibiotic No. 2-200 according to Claim 2, wherein the cultivation is performed at a temperature in the range of from 24 to 32°C for a period of 24 to 144 hours.

5. Pharmaceutical composition comprising an effective amount of antibiotic No. 2-200 according to Claim 1 and a pharmaceutically acceptable carrier and/or diluent and/or auxiliary agent.

## Revendications

1. Antibiotique N° 2-200 de formule:

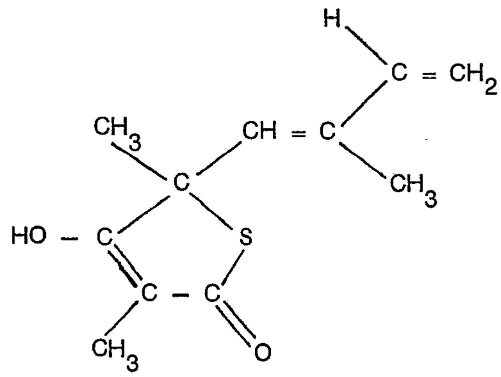

2. Procédé de préparation d'un antibiotique N° 2-200 par culture dans un milieu nutritif du micro-organisme Nocardia sp. N° 2-200, ATCC 31319, produisant l'antibiotique N° 2-200 et récupération de l'antibiotique N° 2-200 accumulé à partir du bouillon de culture.

3. Procédé de préparation d'un antibiotique N° 2-200 selon la revendication 2, dans lequel la culture est réalisée à une température comprise entre 15 et 35°C pendant une période de 12 à 168 heures.

4. Procédé de préparation d'un antibiotique N° 2-200 selon la revendication 2, dans lequel la culture est réalisée à une température comprise entre 24 et 32°C pendant une période de 24 à 144 heures.

5. Composition pharmaceutique comprenant und quantité efficace d'antibiotique N° 2-200 selon la revendication 1 et un véhicule et/ou diluant et/ou agent auxiliaire pharmaceutiquement acceptables.

## Patentansprüche

1. Antibiotikum Nr. 2-200 der Formel

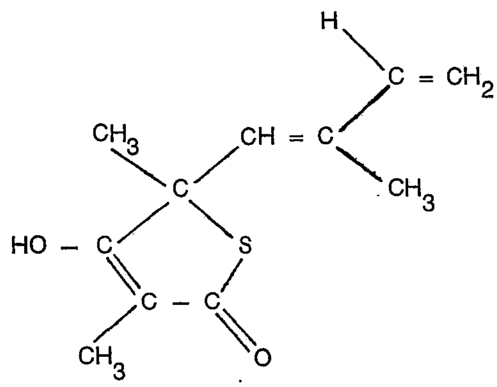

2. Verfahren zur Herstellung eines Antibiotikums Nr. 2-200 durch Züchtung des das Antibiotikum Nr. 2-200 erzeugenden Mikroorganismus Nocardia sp. Nr. 2-200, ATCC 31319 in einem Nährmedium und Gewinnung des angesammelten Antibiotikums Nr. 2-200 aus der Kulturbrühe.

3. Verfahren zur Herstellung eines Antibiotikums Nr. 2-200 gemäss Anspruch 2, wobei die Züchtung bei einer Temperatur im Bereich von 15 bis 35°C innerhalb eines Zeitraumes von 12 bis 168 Stunden durchgeführt wird.

4. Verfahren zur Herstellung eines Antibiotikums Nr. 2-200 gemäss Anspruch 2, wobei die Züchtung bei einer Temperatur im Bereich von 24 bis 32°C während 24 bis 144 Stunden durchgeführt wird.

5. Arzneimittel enthaltend eine wirksame Menge an Antibiotikum Nr. 2-200 gemäss Anspruch 1 und einen pharmazeutisch verträglichen Trägerstoff und/oder ein Verdünnungsmittel und/oder einen Hilfsstoff.

Fig. 1

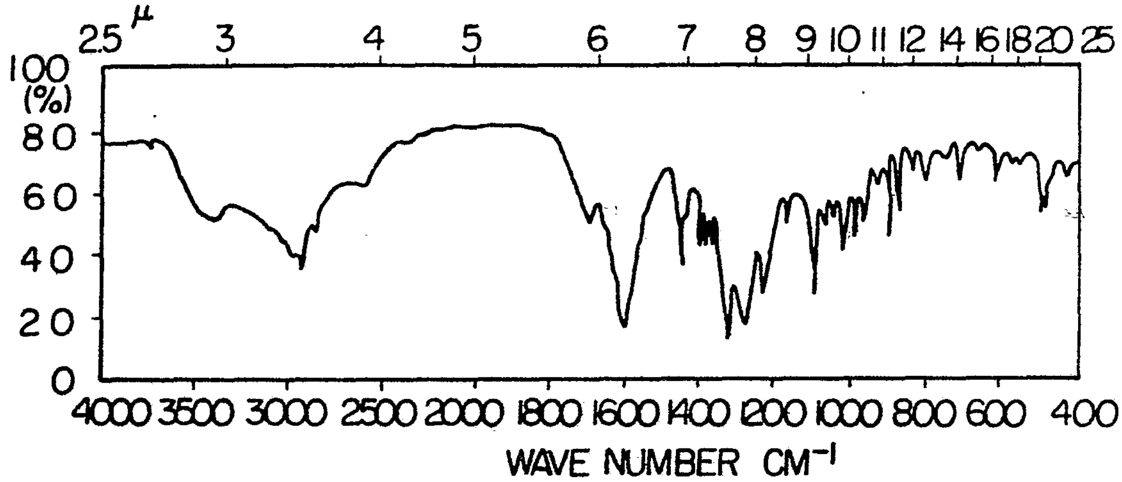

25 μ
3
4
5
6
7
8
9
10
11
12
14
16
18
20
25
100 (%)
80
60
40
20
0
4000 3500 3000 2500 2000 1800 1600 1400 1200 1000 800 600 400
WAVE NUMBER CM⁻¹


Fig. 2

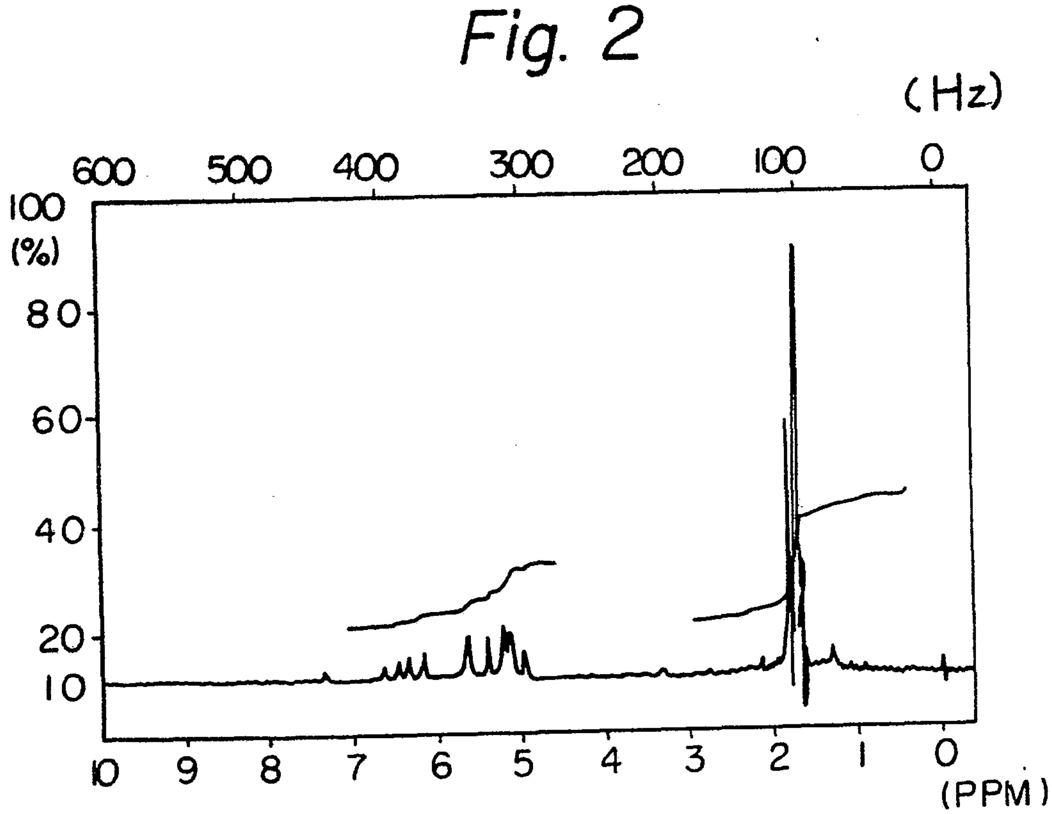

(Hz)
600
500
400
300
200
100
0
100 (%)
80
60
40
20
10
10 9 8 7 6 5 4 3 2 1 0
(PPM)

Fig. 3

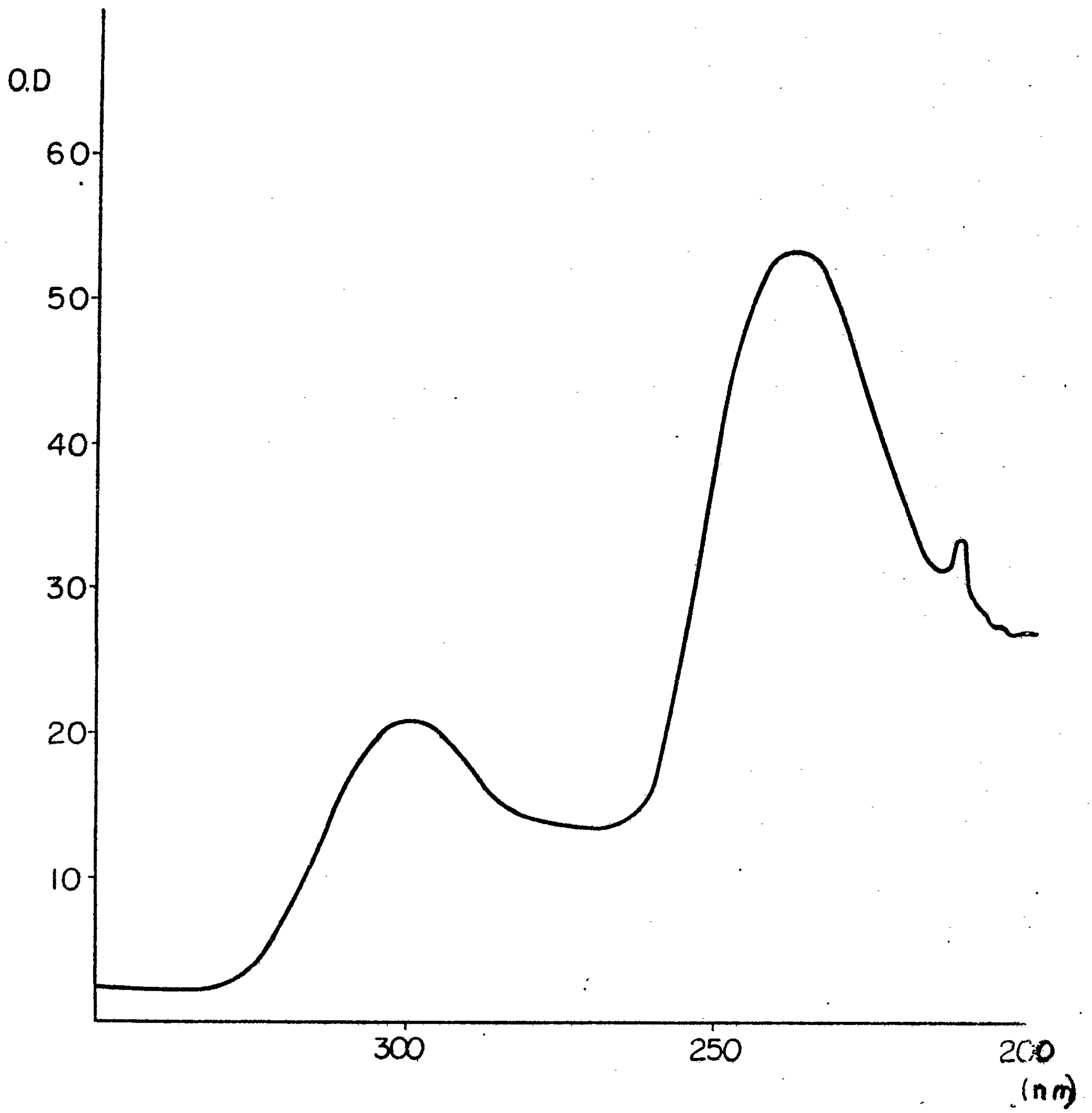

Fig. 4